(19) Europäisches Patentamt
European Patent Office
Office européen des brevets

(11) Publication number: 0 004 570
B1

(12) # EUROPEAN PATENT SPECIFICATION

(45) Date of publication of patent specification: 14.04.82

(21) Application number: 79100700.8

(22) Date of filing: 08.03.79

(51) Int. Cl.³: C 07 D 417/12,
C 07 D 501/36,
A 61 K 31/41

(54) Thiol esters, process for their preparation, pharmaceutical compositions containing them and a process for preparing cephalosporin compounds using the same.

(30) Priority: 09.03.78 JP 25975/78
01.08.78 JP 93121/78

(43) Date of publication of application:
17.10.79 Bulletin 79/21

(45) Publication of the grant of the patent:
14.04.82 Bulletin 82/15

(84) Designated Contracting States:
BE CH DE FR GB LU NL SE

(56) References cited:
US - A - 3 516 997

(73) Proprietor: Asahi Kasei Kogyo Kabushiki Kaisha
2-6, Dojimahama 1-chome Kita-ku
Osaka-shi Osaka 530 (JP)

(72) Inventor: Shibuya, Chisei
100, Kawanarijima
Fuji-shi Shizuoka-ken (JP)
Inventor: Ishii, Kunihiko
100, Kawanarijima
Fuji-shi Shizuoka-ken (JP)
Inventor: Sano, Takumi
845-1, Naganuki Shibakawa-cho
Fuji-shi Shizuoka-ken (JP)
Inventor: Ishida, Torao
100, Kawanarijima
Fuji-shi Shizuoka-ken (JP)
Inventor: Shibukawa, Mitsuru
3-225, Sengencho Nishi-ku
Yokohama-shi Kanagawa-ken (JP)

(74) Representative: Boeters, Hans Dietrich, Dr. et al,
Boeters, Bauer & Partner Thomas-Wimmer-Ring 14
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 004 570 B1

Courier Press, Leamington Spa, England.

# Thiol esters, process for their preparation, pharmaceutical compositions containing them and a process for preparing cephalosporin compounds using the same

This invention relates to novel thiol esters having utility as a sleep-inducing agent, an anti-bacterial agent and an acylating agent for amines and hydrazines, particularly as active esters for preparing cephalosporin compounds, a process for their preparation and a process for the preparation of cephalosporin compounds using the thiol esters.

A 1,3,4-thiadiazole-5-thiol ester of the formula,

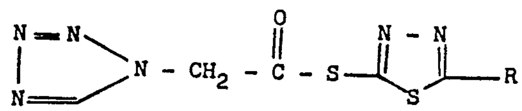

wherein R is a hydrogen atom or a methyl group, and a process for its preparation have not been reported yet.

Further, no method is yet known for preparing a cephalosporin compound of the formula,

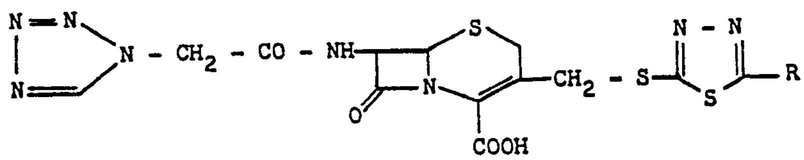

wherein R is a hydrogen atom or a methyl group, in one step using 7-aminocephalosporanic acid or its derivative as the starting material. In order to obtain the above described cephalosporin compound, it is necessary to employ the methods as described in U.S. Patent Nos. 3,278,531 and 3,516,997, Japanese Patent Publication Nos. 5150/1971 and 35751/1971 as a combination. For example, the above described cephalosporin compound may be prepared by two steps, i.e., either by firstly reacting 7-aminocephalosporanic acid with 1H-tetrazole-1-acetic acid and secondly reacting the 7-(1H-tetrazol-1-ylacetamido)-cephalosporanic acid thus obtained with a 1,3,4-thiadiazole-5-thiol or by firstly reacting 7-aminocephalosporanic acid with a 1,3,4-thiadiazole-5-thiol and secondly reacting the 7-amino-3-(1,3,4-thiadiazol-5-yl)-thiomethyl-3-cephem-4-carboxylic acid thus obtained with 1H-tetrazole-1-acetic acid. Thus, these methods require a two-step operation, and further a separation procedure such as an extraction with a solvent for recovering the unstable product formed in the first step and, as a result, the yield is too low for practical purposes.

Accordingly, the present invention in one embodiment provides a 1,3,4-thiadiazole-5-thiol ester of the formula,

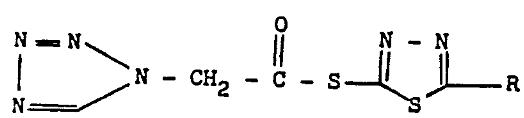 (I)

wherein R is a hydrogen atom or a methyl group.

The present invention in another embodiment provides a process for preparing the above described compound of Formula (I).

In a further embodiment, the invention provides a process for preparing a cephalosporin compound of the formula,

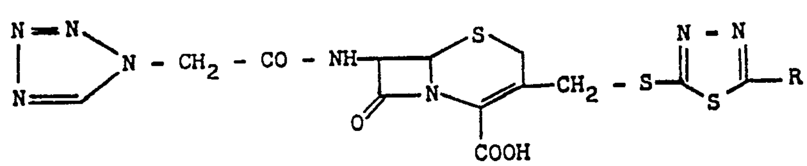 (II)

wherein R is the same as defined above, which comprises reacting 7-aminocephalosporanic acid or its derivative with the above described compound of Formula (I).

The 1,3,4-thiadiazole-5-thiol esters of Formula (I) of this invention are 1H-tetrazole-1-acetic acid 1,3,4-thiadiazol-5-ylthiol ester and 1H-tetrazole-1-acetic acid 2-methyl-1,3,4-thiadiazol-5-ylthiol ester.

These compounds of Formula (I) of this invention are novel compounds and useful as sleep-inducing agents and anti-bacterial agents of high value.

More specifically, the compounds of Formula (I) of this invention have been found to exhibit an anesthetic effect, i.e., a sleep-inducing effect according to the following experiment.

Male ddY-strain mice aged 7 or 6 weeks, each group consisting of 8 animals, were given an intravenous injection of 35 mg/kg of sodium thiopental in an amount corresponding to a certain anesthetic period of time, the period of loss of the writhing reflex being measured as the anesthetic effect. As a test drug, 1H-tetrazole-1-acetic acid 2-methyl-1,3,4-thiadiazol-5-ylthiol ester was dissolved in dimethyl sulfoxide and the test drug thus prepared was orally administered to the animals at a rate of 0.05 ml/10 g weight one hour before the administration of sodium thiopental. The results are shown in the Table below.

Table

| Compound | Amount of Administration (P.O.) (mg/kg) | Period of Time of Anesthesia (Average Value) (minutes) |
|---|---|---|
| 1H-Tetrazole-1-acetic acid 2-methyl-1,3,4-thiadiazol-5-ylthiol ester | 400 | 33.8 |
| " | 200 | 21.0 |
| " | 100 | 10.3 |
| Comparative Group | 0 | 8.4 |

As is clearly to be seen from the Table as described above, the period of time of anesthesia of 1H-tetrazole-1-acetic acid 2-methyl-1,3,4-thiadiazol-5-ylthiol ester used in an amount of 400 mg/kg is 4.02 times greater than that of the comparaive group. Thus, the prolongation effect on the period of time of anesthesia according to the compounds of Formula (I) of this invention is remarkable.

Also, 1H-tetrazole-1-acetic acid 2-methyl-1,3,4-thiadiazol-5-ylthiol ester of this invention had a minimum inhibitory concentration of 50 $\mu$g/mg to *Corynebacterium diphtheriae* P.W. 8 and therefore possesses an antibacterial activity.

Furthermore, the 1,3,4-thiadiazole-5-thiol esters of Formula (I) of this invention are useful as acylating agents for amines and hydrazines, particularly as active esters for preparing cephalosporin compounds.

The 1,3,4-thiadiazole-5-thiol esters of Formula (I) of this invention can be prepared by reacting a 1,3,4-thiadiazole-5-thiol of the formula,

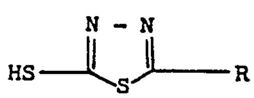

(III)

wherein R is a hydrogen atom or a methyl group, or its derivative with 1H-tetrazole-1-acetic acid or its derivative.

The derivatives of the 1,3,4-thiadiazole-5-thiols which can be employed in this invention include the salts of an alkali metal such as sodium or potassium; the reaction products of and with, respectively, a trialkylaluminum such as trimethylaluminum and triethylaluminum; the silyl derivatives such as the trimethylsilyl derivative and the triethylsilyl derivative; and the trifluoroacetates. The 1,3,4-thiadiazole-5-thiols also exist by tautomerism in the form of the 1,3,4-thiadiazole-5-thiones or as a mixture with the 1,3,4-thiadiazole-5-thiones, and are equivalent to the 1,3,4-thiadiazole-5-thiones or the mixture which are accordingly included, as equivalents, in the 1,3,4-thiadiazole-5-thiols of this invention.

The derivatives of the carboxylic group in the 1H-tetrazole-1-acetic acid which can be employed in this invention include the acid halides, the acid anhydride, the mixed acid anhydrides, the acid amides, the esters, the acid azides and the nitrile.

Specific examples of suitable derivatives include the acid chloride; the mixed acid anhydrides of and with, respectively, a dialkylphosphoric acid such as dimethylphosphoric acid and diethylphosphoric acid, phenylphosphoric acid, diphenylphosphoric acid, dibenzylphosphoric acid, a halogenated phosphoric acid such as chlorophosphoric acid and bromophosphoric acid, a dialkylphosphorous acid such as dimethylphosphorous acid and diethylphosphorous acid, sulfurous acid, thiosulfuric acid, sulfuric acid, an alkylcarbonic acid such as methylcarbonic acid and ethylcarbonic acid, an aliphatic carboxylic acid such as pivalic acid, pentanoic acid, isopentanoic acid, 2-ethylbutanoic acid, trichloroacetic acid and trifluoroacetic acid, an aromatic carboxylic acid such as benzoic acid or the symmetric acid (and symmetric acid anhydride, respectively) of the 1H-tetrazole-1-acetic acid; the acic amides of and with, respectively, imidazoles, a 4-substituted imidazole such as 4-methylimidazole and 4-ethylimidazole, dimethylpyrazole, triazole, tetrazole, ammonia, methylamine, dimethylamine; the esters such as methyl ester, ethyl ester, cyanomethyl ester, methoxymethyl ester, vinyl ester, propargyl ester, p-nitrophenyl ester, 2,4-dinitrophenyl ester, trichlorophenyl ester, pentachlorophenyl ester, methanesulfonylphenyl ester, phenylazophenyl ester, phenyl ester, phenylthioester, p-nitrophenylthioester, 2,4-dinitrophenylthioester, p-cresylthioester, carboxymethylthioester, pyranyl ester, pyridyl ester, 8-quinolylthioester, the ester of and with, respectively, N,N-dimethylhydroxylamine, 1-hydroxy-2-(1H)-pyridone, N-hydroxysuccinimide or N-hydroxyphthalimide.

The amount of the 1,3,4-thiadiazole-5-thiol of Formula (III) or its derivative which can be employed for the preparation of the 1,3,4-thiadiazole-5-thiol esters of Formula (I) of this invention typically ranges from 0.5 moles to 2 moles, and preferably from 0.9 moles to 1.2 moles, per mole of 1H-tetrazole-1-acetic acid or its derivative.

When free 1H-tetrazole-1-acetic acid is used, it is necessary to employ a dehydrating agent for condensation.

Appropriate dehydrating agents for condensation which can be employed in this invention include N,N-dicyclohexylcarbodiimide, N,N'-diisopropylcarbodiimide, N-cyclohexyl-N'-morpholinoethylcarbodiimide, N-cyclohexyl-N'-(4-diethylaminocyclohexyl)carbodiimide, N,N'-diethylcarbodiimide, N-ethyl-N'-(3-dimethylaminopropyl) carbodiimide, N,N'-carbonyldi(2-methylimidazole), pentamethyleneketene-N-cyclohexylimine, diphenylketene-N-cyclohexylimine, an alkoxyacetylene such as methoxyacetylene and ethoxyacetylene, an 1-alkoxy-1-chloroethylene such as 1-methoxy-1-chloroethylene and 1-ethoxy-1-chloroethylene, a trialkyl phosphite such as trimethyl phosphite and triethyl phosphite, ethyl polyphosphates, isopropyl polyphosphate, phosphorus oxychloride, thionyl chloride, oxalyl chloride, triphenylphosphine, 2-ethyl-7-hydroxybenzisoxazolium salt, 2-ethyl-5-m-sulfophenylisoxazolium hydroxide inner salt, (chloromethylene) dimethylammonium chloride, $SO_3$-hexamethylphosphoramide, $SO_3$-N,N-dimethylformamide, sulfonates, triflouroacetic anhydride, diphenylphosphoric acid azide, diphenylphosphoric acid cyanide and any mixtures thereof.

The amount of the dehydrating agent for condensation is typically 0.5 mole to 20 moles, and preferably 1 mole to 10 moles, per mole of free 1H-tetrazole-1-acetic acid.

The reaction temperature which can be employed in the preparation of the 1,3,4-thiadiazole-5-thiol esters of Formula (I) may be varied within a wide range of temperatures. In general, the reaction temperature is −50°C to 100°C, and preferably −20°C to 50°C.

The reaction of this invention can be conducted, in general, at atmospheric pressure, but it should be noted that the pressure employed is not limited thereto. Generally the reaction is conducted in an atmosphere of air. However, it may also be conducted in an atmosphere of an inert gas such as nitrogen, argon and helium.

This reaction is generally conducted in a reaction medium. Any reaction medium which is inactive to the reactants and can dissolve or suspend the reactants can be employed in this invention.

Specific examples of suitable reaction media include dioxane, ethyl acetate, methylene chloride, chloroform, hexane, trifluoroacetic anhydride and aqueous solutions of sodium hydroxide or potassium hydroxide.

The amount of 1H-tetrazole-1-acetic acid or its derivative which can be employed is typically 1 g to 50 g, and preferably 5 g to 20 g, per 100 ml of the reaction medium.

After the reaction is substantially completed, the desired compounds of Formula (I) can be recovered from the reaction mixture and purified by the conventional techniques. For example, the reaction medium is evaporated from the reaction mixture and the crude product thus obtained is washed, dried or recrystallized.

The cephalosporin compounds of Formula (II) of this invention can be prepared by reacting 7-aminocephalosporanic acid or its derivative with the 1,3,4-thiadiazole-5-thiol ester of Formula (I).

According to the present process for the preparation of the cephalosporin compounds of Formula (II), the acylation of the 7-position in the 7-aminocephalosporanic acid or its derivative and the substitution of the 3-position in the 7-aminocephalosporanic acid or its derivative proceed selectively in one step to give the desired compounds of Formula (II). Thus, the reaction operation is simple and the recovery of the desired compounds of Formula (II) can be simplified. As a result, the desired compounds of Formula (II) having high purity can be obtained at high yields.

Suitable examples of the derivatives of 7-aminocephalosporanic acid which can be employed in this invention include the salts of alkali metals such as sodium and potassium; the salts of alkaline earth metals such as calcium and magnesium; the salts of nitrogen-containing organic bases such as trimethylamine, triethylamine, pyridine, N-methylpiperidine, N-methylmorpholine; and the esters which can be easily released by catalytic reduction or chemical reduction or under other mild conditions, such as the toluenesulfonylethyl ester, benzyl ester, p-nitrobenzyl ester, phenacyl ester, diphenylmethyl ester, trityl ester, t-butyl ester, an alkyloxymethyl ester such as methoxymethyl ester and ethoxymethyl

ester, phenyloxymethyl ester, benzoyloxymethyl ester, acetyloxymethyl ester, 3,5-di (t-butyl)-4-hydroxybenzyl ester, and $\beta,\beta,\beta$-trichloroethyl ester.

The amount of the 1,3,4-thiadiazole-5-thiol ester of Formula (I) which can be employed in the preparation of the cephalosporin compounds of Formula (II) is typically 0.5 mole to 2.0 moles, and preferably 0.9 mole to 1.2 moles, per mole of 7-aminocephalosporanic acid or its derivative.

The preparation of the cephalosporin compounds of Formula (II) is generally conducted in a reaction medium. Any reaction medium which is inactive to the reactants can be employed in this invention.

Specific examples of suitable reaction media used include water, acetone, dioxane, acetonitrile, toluene, chloroform, methylene chloride, ethylene chloride, tetrahydrofuran, ethyl acetate, formic acid, pyridine, trifluoroacetic acid, N,N-dimethylformamide, methanol, ethanol, methoxy ethanol, diethyl ether, isopropyl ether, N,N-dimethylacetamide, dimethyl sulfoxide and any mixtures of water with the above described organic reaction media.

The amount of 7-aminocephalosporanic acid or its derivative which can be employed is typically 1 g to 50 g and preferably 5 g to 20 g per 100 ml of the reaction medium.

It is preferred that the reaction between 7-aminocephalosporanic acid or its derivative and the 1,3,4-thiadiazole-5-thiol ester of Formula (I) be conducted at a pH ranging from 1 to 9. A more preferred pH is 3 to 8.

The temperature of the above described reaction which can be employed in this invention is −50°C to 100°C, and preferably 0°C to 80°C. In general, the above described reaction is carried out at atmospheric pressure, but it should be noted that the pressure employed is not limited thereto. The above described reaction may be carried out in an atmosphere of air, and preferably in an atmosphere of an inert gas such as nitrogen, argon and helium.

The above described reaction is continued until the desired compound of Formula (II) is produced in a most appropriate amount. The period of time of the above described reaction typically ranges from 10 minutes to several tens of hours, and preferably from 0.5 hour to 5 hours.

Further, in order to promote the above described reaction of this invention there can be employed a base including an alkali metal hydrogencarbonate such as sodium hydrogencarbonate and potassium hydrogencarbonate, a trialkylamine such as trimethylamine and triethylamine, pyridine; an inorganic acid salt such as a chloride, bromide, iodide, thiocyanate or nitrate of lithium, sodium, potassium or ammonium; a metal compound such as cupric chloride, cupric bromide, cupric fluoride, cupric nitrate, cuprous nitrate, cupric sulfate, cuprous sulfate, cupric borate, copper metaborate, cupric phosphate, cupric cyanide, cuprous cyanide, cupric formate, cuprous formate, cupric acetate, cuprous acetate, copper propionate, copper citrate, cupric tartrate, cuprous tartrate, cupric benzoate, cuprous benzoate, cupric salicylate, cuprous salicylate, ferric chloride, ferrous chloride; a Lewis acid such as titanium tetrachloride, titanium tetrabromide, zirconium tetrachloride, silicon tetrachloride, tin tetrachloride, antimony trichloride, antimony pentachloride, bismuth trichloride, aluminum trichloride, aluminum tribromide, zinc dichloride, boron trifluoride, boron trichloride, boron tribromide; a quaternary ammonium salt such as tetramethylammonium chloride, tetraethylammonium bromide, dimethyl-diphenylammonium chloride, triethylbenzylammonium bromide; and a tetraalkylphosphonium halide such as tetramethylphosphonium chloride, tetraethylphosphonium bromide, and tetraphenylphosphonium iodide.

The amount of these compounds which can be employed to promote the above-described reaction typically ranges from 0.1 mole to 10 moles, and preferably from 1 mole to 5 moles, per mole of 7-aminocephalosporanic acid or its derivative.

The reaction products are separated and collected by the conventional methods from the reaction mixture and the cephalosporin compounds of Formula (II) of this invention can be isolated either as such or in the form of the salts or the esters. When the cephalosporin compounds of Formula (II) are obtained in the form of the esters, the esters can be converted into the cephalosporin compounds by catalytic reduction or chemical reduction or under other mild conditions.

The cephalosporin compounds of Formula (II) thus obtained can be converted, by the conventional methods into the salts of an alkali metal such as sodium or potassium; the salt of ammonium; the salts of an alkaline earth metal such as magnesium or calcium; and the salts of diphenylenediamine, dicyclohexylamine, dibenzylethylenediamine, triethylamine, tri-n-butylamine, triphenylamine, triallylamine, dibenzylamine, N,N-dibenzylaminoethanol, procaine, quinine, 2-methylquinoline, 2-amino-5-nitrothiazole, 9-aminoacridine or guanylurea. These salts exhibit excellent features for preparing medicaments, for example, from the view-point of their water solubility.

The cephalosporin compounds of Formula (II) are useful as antimicrobial agents.

The following Examples are given to illustrate the present invention more specifically. However, it should be understood that the invention is in no way limited to these Examples.

Example 1

1.3 g of 1H-tetrazole-1-acetic acid were suspended in 20 ml of trifluoroacetic anhydride and stirred at 20°C for 15 minutes. Then the trifluoroacetic anhydride and by-products were distilled off under reduced pressure, and a mixed solution of 1.3 g of 2-methyl 1,3,4-thiadiazole-5-thiol and 50 ml

of ethyl acetate was added to the residue and stirred at 20°C for 3 hours. The solution thus obtained was concentrated to dryness to give 3.8 g of a solid, and the solid was dissolved in a small amount of ethyl acetate, and then 20 ml of petroleum ether were added thereto. The precipitate thus formed was collected by filtration and dried to give 2.40 g of 1H-tetrazole-1-acetic acid 2-methyl-1,3,4-thiadiazol-5-ylthiol ester in the form of yellowish powder at a yield of 99%.

Elemental Analysis Values:
Calculated (%): C, 29.8; H, 2.5; N, 34.7; S, 26.4
Found (%): C, 29.9; H, 2.5; N, 34.5; S, 26.6
Infrared Absorption Spectrum:
$ny_{c=o}$: 1762 $cm^{-1}$
NMR Spectrum ($CDCl_3$), delta ppm:
2.58 (s, 3H), 5.95 (s, 2H), 8.95 (s, 1H)
Ultraviolet Absorption Spectrum (in ethyl acetate):
$lambda_{max} = 323$ nm

Example 2

The same procedure as in Example 1 was repeated except that 1.2 g of 1,3,4-thiadiazole-5-thiol were used instead of the 1.3 g of 2-methyl-1,3,4-thiadiazole-5-thiol. As a result, 2.2 g of 1H-tetrazole-1-acetic acid 1,3,4-thiadiazol-5-ylthiol ester were obtained in the form of yellowish powder.

Elemental Analysis Values:
Calculated (%): C, 26.3; H, 1.8; N, 36.8; S, 28.1
Found (%): C, 26.5; H, 1.8; N, 36.5; S, 28.3
Infrared Absorption Spectrum:
$ny_{c=o}$: 1765 $cm^{-1}$

Example 3

1.3 g of 2-methyl-1,3,4-thiazole-5-thiol were dissolved in 100 ml of ethyl acetate and then 1.3 g of 1H-tetrazole-1-acetic acid with stirring, followed by a further addition of 2.06 g of dicyclohexylcarbodiimide with stirring under cooling with ice. The mixture was stirred under cooling with ice for 4 hours and left to stand at 0°C for 20 hours. Then the precipitate of dicyclohexylurea was separated by filtration and the filtrate was washed twice with 30 ml of saturated sodium chloride aqueous solution and dehydrated with anhydrous magnesium sulfate, and subsequently ethyl acetate was distilled at 25°C under reduced pressure. The residue was dissolved in a small amount of ethyl acetate, and the addition of 20 ml of petroleum ether thereto resulted in the formation of precipitates. The precipitates were collected by filtration and dried to give 2.26 g of 1H-tetrazole-1-acetic acid 2-methyl-1,3,4-thiadiazol-5-ylthiol ester at a yield of 93.4%. The analytical values of this product were the same as those of Example 1.

Example 4

8 ml of trimethylaluminium solution having 2.5 mol concentration in hexane were dissolved in 20 ml of methylene chloride and cooled to 0°C. 2.6 g of 2-methyl-1,3,4-thiadiazole-5-thiol were added to the solution at 0°C and the reaction was continued at 25°C for 2.5 hours in a nitrogen atmosphere with stirring. Then 1.4 g of 1H-tetrazole-1-acetic acid methyl ester were added to the solution and the reaction was continued at 25°C for 6 hours with stirring. The reaction medium and unreacted substances were distilled from the reaction solution under reduced pressure and 50 ml of ethyl acetate were added to the residue thus obtained and insoluble materials were removed by filtration. The addition of 50 ml of petroleum ether to the filtrate resulted in the precipitation of solids and the solids were collected by filtration and dried to give 2.31 g of 1H-tetrazole-acetic acid 2-methyl-1,3,4-thiadiazol-5-ylthiol ester at a yield of 95%. The analytical values of the product were the same as those of Example 1.

Example 5

The same procedure as in Example 4 was repeated except that 1.56 g of 1H-tetrazole-1-acetic acid ethyl ester were used instead of the 1.4 g of 1H-tetrazole-1-acetic acid methyl ester. As a result, 2.35 g of the same 1H-tetrazole-1-acetic acid 2-methyl-1,3,4-thiazol-5-ylthiol ester as in Example 4 were obtained at a yield of 97%.

Example 6

A solution of 1.2 g of the 1H-tetrazole-1-acetic acid 2-methyl-1,3,4-thiadiazol-5-ylthiol ester as obtained in Example 1 and 10 ml of acetone was added to a solution of 1.4 g of 7-aminocephalosporanic acid, 0.4 g of sodium bicarbonate and 60 ml of water with stirring. The mixed solution thus obtained was further stirred at 60°C for 4 hours in a nitrogen atmosphere, the pH of the solution being adjusted to 6.4 with a 3% sodium bicarbonate aqueous solution. After completion of the reaction

acetone was distilled from the reaction solution under reduced pressure, and the water layer was washed twice with 50 ml of ether. Then the pH of the water layer was adjusted to 3 with 3N hydrochloric acid and the water layer was extracted three times with 100 ml of ethyl acetate. Then the extract was dried over sodium sulfate, and the reaction medium was distilled to give 2.2 g of 7 - (1H - tetrazol - 1 - ylacetamido) - 3 - (2 - methyl - 1,3,4 - thiadiazol - 5 - ylthiomethyl) - 3 - cephem - 4 - carboxylic acid having a purity of 93.5% at a yield of 90.6%.

Ultraviolet Absorption Spectrum (in 3% sodium hydrogencarbonate aqueous solution):
$lambda_{max} = 272$ nm

1.44 g of the above described cephalosporin compound were dissolved in 100 ml of an aqueous solution containing 0.1 M sodium acetate and 0.2 M sodium chloride and the pH of the solution was adjusted to 4.5 with acetic acid. The solution thus prepared was poured in a 150 ml column packed with Amberlite XAD—2 (manufactured by Rohm & Haas Co., Ltd.) to adsorb the desired product thereon. After being washed with 3 l of a 5% acetic acid aqueous solution and further with 750 ml of water, the product was dissolved in a 50% acetone aqueous solution and the solution containing the desired product was freeze-dried to give 1.22 g of purified 7 - (1H - tetrazol - 1 - ylacetamido) - 3 - (2 - methyl - 1,3,4 - thiadiazol - 5 - ylthiomethyl) - 3 - cephem - 4 - carboxylic acid having a purity of 99.1%.

Ultraviolet Absorption Spectrum (in 3% sodium bicarbonate aqueous solution):
$lambda_{max} = 272$nm

Melting Point: 197°C—200°C (Decomp.)

NMR Spectrum (DMSO-$d_6$), delta ppm:
2.68 (s, 3H), 3.71 (dd, 2H), 4.39 (dd, 2H), 5.12 (d, 1H), 5.37 (s, 2H), 5.73 (q, 1H), 9.33 (s, 1H), 9.49 (d, 2H).

0.92 g of the compound having a purity of 99.1% thus obtained and 0.17 g of sodium bicarbonate were dissolved in 2 ml of water and the solution was filtered. 9 ml of 99% ethanol were added to the filtrate and a crystal of sodium 7 - (1H - tetrazol - 1 - ylacetamido) - 3 - (2 - methyl - 1,3,4 - thiadiazol - 5 - ylthiomethyl) - 3 - cephem - 4 - carboxylate was collected by filtration.

Ultraviolet Absorption Spectrum (in 3% sodium bicarbonate aqueous solution):
$lambda_{max} = 272$ nm
NMR Spectrum (DMSO-$d_6$), delta ppm:
2.70 (s, 3H), 3.67 (dd, 2H), 4.57 (dd, 2H), 5.08 (d, 1H), 5.47 (s, 2H), 5.65 (q, 1H), 9.50 (s, 1H), 9.72 (d, 2H)

## Example 7

The same procedure as in Example 6 was repeated except that 1.1 g of the 1H-tetrazole-1-acetic acid 1,3,4-thiadiazol-5-ylthiol ester as obtained in Example 2 were used instead of the 1.2 g of 1H-tetrazole-1-acetic acid 2-methyl 1,3,4-thiadiazol-5-ylthiol ester. As a result, 2.1 g of 7 - (1H - tetrazol - 1 - ylacetamido) - 3 - (1,3,4 - thiadiazol - 5 - ylthiomethyl) - 3 - cephem - 4 - carboxylic acid having a purity of 93.1% were obtained at a yield of 88.9%.

1 g of this compound was recrystallized from a 50% acetone aqueous solution to give 0.55 g of a purified crystal having a melting point of 155°C (decomp.) and a purity of 98.5%.

Ultraviolet Absorption Spectrum (3% sodium hydrogencarbonate aqueous solution):
$lambda_{max} = 273$ nm

## Example 8

A solution of 1.2 g of 1H-tetrazole-1-acetic acid 2-methyl-1,3,4-thiadiazol-5-ylthiol ester, 5 ml of N,N-dimethylformamide and 5 ml of water was added to a solution of 1.4 g of 7-aminocephalosporanic acid, 30 ml of N,N-dimethylformamide and 30 ml of water with stirring and the mixed solution thus obtained was further stirred at 60°C for 4 hours in a nitrogen atmosphere. After completion of the reaction the N,N-dimethylformamide and water were distilled from the reaction solution and 50 ml of water and 100 ml of ethyl acetate were added to the residue. The pH of the solution thus obtained was adjusted to 3 with 3N hydrochloric acid and then the solution was extracted with ethyl acetate and further twice with 100 ml of ethyl acetate. The extract was dried over sodium sulfate and the ethyl acetate was distilled to give 2.16 g of 7 - (1H - tetrazol - 1 - ylacetamido) - 3 - (2 - methyl - 1,3,4 - thiadiazol - 5 - ylthiomethyl) - 3 - cephem - 4 - carboxylic acid having a purity of 92.5% at a yield of 89.0%. The analytical values of this product were found to be in agreement with those of the product in Example 6.

## Claims

1. A 1,3,4-thiadiazole-5-thiol ester of the formula,

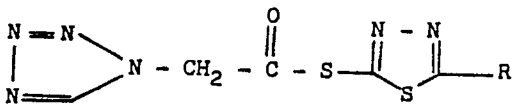

wherein R is a hydrogen atom or a methyl group.

2. A process for preparing the 1,3,4-thiadiazole-5-thiol ester of Claim 1 which comprises reacting a 1,3,4-thiadiazole-5-thiol of the formula,

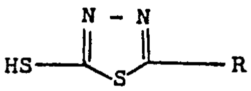

wherein R is a hydrogen atom or a methyl group, or its derivative with 1H-tetrazole-1-acetic acid (in the presence of a dehydrating agent) or its derivative.

3. A process according to Claim 2, characterized in that a derivative of the 1,3,4-thiadiazole-thiol is used which is selected from the following group:
the salts of an alkali metal such as sodium or potassium; the reaction products with a trialkylaluminum such as trimethylaluminum and triethylaluminum; the silyl derivatives such as the trimethylsilyl derivative and the triethylsilyl derivative; and the trifluoroacetates.

4. A process according to claim 2 or 3, characterized in that a derivative of the 1H-tetrazole-1-acetic acid is used which is selected from the following group:
the acid halides such as the acid chloride; the mixed acid anhydrides with a dialkylphosphoric acid such as dimethylphosphoric acid and diethylphosphoric acid, phenylphosphoric acid, diphenylphosphoric acid, dibenzylphosphoric acid, a halogenated phosphoric acid such as chlorophosphoric acid and bromophosphoric acid, a dialkylphosphorous acid such as dimethylphosphorous acid and diethylphosphorous acid, sulfurous acid, thiosulfuric acid, sulfuric acid, an alkylcarbonic acid such as methylcarbonic acid and ethylcarbonic acid, an aliphatic carboxylic acid such as pivalic acid, pentanoic acid, isopentanoic acid, 2-ethylbutanoic acid, trichloroacetic acid and trifluoroacetic acid, an aromatic carboxylic acid such as benzoic acid; the symmetric acid anhydride of the 1H-tetrazole-1-acetic acid; the acid amides with imidazole, a 4-substituted imidazole such as 4-methylimidazole and 4-ethylimidazole, dimethylpyrazole, triazole, tetrazole, ammonia, methylamine, dimethylamine; the esters such as methyl ester, ethyl ester, cyanomethyl ester, methoxymethyl ester, vinyl ester, propargyl ester, p-nitrophenyl ester, 2,4 dinitrophenyl ester, trichlorophenyl ester, pentachlorophenyl ester, methanesulfonylphenyl ester, phenylazophenyl ester, phenyl ester, phenylthioester, p-nitrophenylthioester, 2,4-dinitrophenylthioester, p-cresylthioester, carboxymethylthioester, pyranyl ester, pyridyl ester, 8-quinolylthioester, the ester with N,N-dimethylhydroxylamine, 1-hydroxy-2-(1H)-pyridone, N-hydroxysuccinimide or N-hydroxyphthalimide; the acid azides; and the nitrile.

5. A process according to claim 2 or 3, characterized in that the free 1H-tetrazole-1-acetic acid is used in the presence of a dehydrating agent which is selected from the following group:
N,N-dicyclohexylcarbodiimide, N,N'/diisopropylcarbodiimide, N-cyclohexyl-N'-morpholinoethylcarbodiimide, N-cyclohexyl-N'-(4-diethylaminocyclohexyl) carbodiimide, N,N'-diethylcarbodiimide, N-ethyl-N'-(3-dimethylaminopropyl) carbodiimide, N,N'-carbonyldi(2-methylimidazole), pentamethyleneketenes-N-cyclohexylimine, diphenylketene-N-cyclohexylimine, an alkoxyacetylene such as methoxyacetylene and ethoxyacetylene, an 1-alkoxy-1-chloroethylene such as 1-methoxy-1-chloroethylene and 1-ethoxy-1-chloroethylene, a trialkyl phosphite such as trimethyl phosphite and triethyl phosphite, ethyl polyphosphates, isopropyl polyphosphate, phosphorous oxychloride, thionyl chloride, oxalyl chloride, triphenylphosphine, 2-ethyl-7-hydroxybenzisoxazolium salt, 2-ethyl-5-m-sulfophenylisoxazolium hydroxide inner salt, (chlormethylene)dimethylammonium chloride, $SO_3$-hexamethylphosphoramide, $SO_3$-N,N-dimethylformamide, sulfonates, trifluoroacetic anhydride, diphenylphosphoric acid azide, diphenylphosphoric acid cyanide and any mixtures thereof; where the said dehydrating agent is used in an amount of 0.5 mole to 20 moles, and preferably 1 mole to 10 moles, per mole of free 1H-tetrazole-1-acetic acid.

6. A process according to claim 2, 3, 4 or 5, characterized in that the 1,3,4-thiadiazole-5-thiol or its derivative is used in an amount in the range of 0.5 moles to 2 moles, and preferably 0.9 moles to 1.2 moles, per mole of 1H-tetrazole-1-acetic acid or its derivative.

7. A process according to claim 2, 3, 4, 5 or 6, characterized in that the reaction is carried out at a reaction temperature in the range of −50°C to 100°C, and preferably −20°C to 50°C.

8. A process according to claim 2, 3, 4, 5, 6 or 7, characterized in that the reaction is carried out in an inactive reaction medium.

9. A process for preparing a cephalosporin compound of the formula,

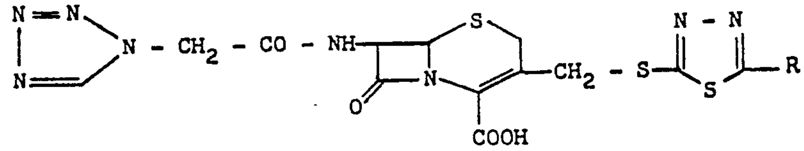

wherein R is a hydrogen atom or a methyl group, which comprises reacting 7-aminocephalosporanic acid or its derivative with the 1,3,4-thiadiazole-5-thiol ester of Claim 1 at a temperature of —50°C to 100°C and preferably 0°C to 80°C.

10. A process according to Claim 9 characterized in that a derivative of 7-aminocephalosporanic acid is used which is selected from the following group:
the salts of alkali metals such as sodium and potassium; the salts of alkaline earth metals such as calcium and magnesium; the salts of nitrogen-containing organic bases such as trimethylamine, triethylamine, pyridine, N-methylpiperidine, N-methylmorpholine; and the esters which can be easily released by catalytic reduction or chemical reduction or under other mild conditions, such as the toluenesulfonylethyl ester, benzyl ester, p-nitrobenzyl ester, phenacyl ester, diphenylmethyl ester, trityl ester, t-butyl ester, an alkyloxymethyl ester such as methoxymethyl ester and ethoxymethyl ester, phenyloxymethyl ester, benzoyloxymethyl ester, acetyloxymethyl ester, 3,5-di-(t-butyl)-4-hydroxybenzyl ester, and β,β,β-trichloroethyl ester.

11. A process according to claim 9 or 10, characterized in that the 1,3,4-thiadiazole-5-thiol ester is used in an amount in the range of 0.5 mole to 2.0 moles, and preferably 0.9 mole to 1.2 moles, per mole of 7-aminocephalosporanic acid or its derivative.

12. A process according to claim 9, 10 or 11, characterized in that the reaction is carried out in an inactive reaction medium.

13. A process according to claim 9, 10, 11 or 12, characterized in that the reaction is carried out at a pH in the range of 1 to 9 and preferably 3 to 8.

14. A process according to claim 9, 10, 11, 12 or 13, characterized in that the reaction is carried out in the presence of a promotor which is selected from the following group:
a base including an alkali metal hydrogencarbonate such as sodium hydrogencarbonate and potassium hydrogencarbonate, a trialkylamine such as trimethylamine and triethylamine, pyridine; an inorganic acid salt such as a chloride, bromide, iodide, thiocyanate or nitrate of lithium, sodium, potassium or ammonium; a metal compound such as cupric chloride, cupric bromide, cupric fluoride, cupric nitrate, cuprous nitrate, cupric sulfate, cuprous sulfate, cupric borate, copper metaborate, cupric phosphate, cupric cyanide, cuprous cyanide, cupric formate, cuprous formate, cupric acetate, cuprous acetate, copper propionate, copper citrate, cupric tartrate, cuprous tartrate, cupric benzoate, cuprous benzoate, cupric salicylate, cuprous salicylate, ferric chloride, ferrous chloride; a Lewis acid such as titanium tetrachloride, titanium tetrabromide, zirconium tetrachloride, silicon tetrachloride, tin tetrachloride, antimony trichloride, antimony pentachloride, bismuth trichloride, aluminum trichloride, aluminum tribromide, zinc dichloride, boron trifluoroide, boron trichloride, boron tribromide; a quaternary ammonium salt such as tetramethylammonium chloride, tetraethylammonium bromide, dimethyldiphenylammonium chloride, triethylbenzylammonium bromide; and a tetraalkylphosphonium halide such as tetramethylphosphonium chloride, tetraethylphosphonium bromide, and tetraphenylphosphonium iodide; where the said promotor is used in an amount in the range of 0.1 mole to 10 moles, and preferably from 1 mole to 5 moles, per mole of 7-aminocephalosporanic acid or its derivative.

15. Composition for sleep-inducing anesthetic, or anti-bacterial purposes, characterized in that it consists of or contains a 1,3,4-thiadiazole-5-thiol ester according to Claim 1.

**Patentansprüche**

1. Ein 1,3,4-Thiadiazol-5-thiolester der Formel

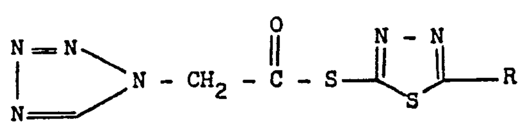

worin R ein Wasserstoffatom oder ein Methylrest ist.

2. Verfahren zur Herstellung des 1,3,4-Thiadiazol-5-thiol-esters gemäß Anspruch 1, dadurch gekennzeichnet, daß man ein 1,3,4-Thiadiazol-5-thiol der Formel

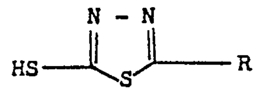

worin R ein Wasserstoffatom oder ein Methylrest ist, oder sein Derivat mit 1H-Tetrazol-1-essigsäure (in Gegenwart eines Dehydratisierungsmittels) oder seines Derivates umsetzt.

3. Verfahren nach Anspruch 2, dadurch gekennzeichnet, daß man ein Derivat des 1,3,4-Thiadiazolthiols verwendet, das aus der nachstehenden Gruppe ausgewählt ist:
die Salze eines Alkalimetalls, wie z.B. natrium oder kalium; die Reaktionsprodukte mit einem Trialkylaluminium, wie z.B. Trimethylaluminium und Triäthylaluminium; die Silylderivate, wie z.B. das Trimethylsilylderivat und das Triäthylsilylderivat; und die Trifluoroacetate.

4. Verfahren nach Anspruch 2 oder 3, dadurch gekennzeichnet, daß man ein Derivat der 1H-Tetrazol-1-essigsäure verwendet, das aus der nachstehenden Gruppe ausgewählt ist:
die Säurehalogenide, wie z.B. das Säurechlorid; die gemischten Säureanhydride mit einer Dialkylphosphorsäure, wie z.B. Dimethylphosphorsäure und Diäthylphosphorsäure, Phenylphosphorsäure, Diphenylphosphorsäure, Dibenzylphosphorsäure, einer halogenierten Phosphorsäure, wie z.B. Chlorphosphorsäure und Bromphosphorsäure, einer Dialkylphosphorigensäure, wie z.B. Dimethylphosphorigensäure, Diäthylphosphorigensäure, Schwefeligersäure, Thioschwefelsäure, Schwefelsäure, einer Alkylkohlensäure, wie z.B. Methylkohlensäure und Äthylkohlensäure, einer aliphatischen Carbonsäure, wie z.B. Pivalinsäure, Valeriansäure, Isovaleriansäure, 2-Äthylbuttersäure, Trichloressigsäure und Trifluoressigsäure, einer aromatischen Carbonsäure, wie z.B. Benzoesäure; das symmetrische Säureanhydrid der 1H-Tetrazol-1-essigsäure; die Säureamide mit Imidazol, einem 4-substituierten Imidazol, wie z.B. 4-Methylimidazol und 4-Äthylimidazol, Dimethylpyrazol, Triazol, Tetrazol, Ammoniak, Methylamin, Dimethylamin; die Ester, wie z.B. Methylester, Äthylester, Cyanomethylester, Methoxymethylester, Vinylester, Propargylester p-Nitrophenylester, 2,4-Dinitrophenylester, Trichlorophenylester, Pentachlorphenylester, Methansulfonylphenylester, Phenylazophenylester, Phenylester, Phenylthioester, p-Nitrophenylthioester, 2,4-Dinitrophenylthioester, p-Cresylthioester, Carboxymethylthioester, Pyranylester, Pyridylester, 8-Chinolylthioester, die Ester mit N,N-Dimethylhydroxylamin, 1-Hydroxy-2-(1H)-pyridon, N-Hydroxysuccinimid oder N-Hydroxyphthalimid; die Säureazide; und das Nitril.

5. Verfahren nach Anspruch 2 oder 3, dadurch gekennzeichnet, daß man die freie 1H-Tetrazol-1-essigsäure in Gegenwart eines Dehydratisierungsmittels verwendet, das aus der nachstehenden Gruppe ausgewählt ist:
N,N - Dicyclohexylcarbodiimid, N,N′ - Diisopropylcarbodiimid, N,N′ - Diisopropylcarbodiimid, N - Cyclohexyl - N′ - morpholinoäthylcarbodiimid, N - Cyclohexyl - N′ - (4 - diäthylaminocyclohexyl)-carbodiimid, N,N′-Diäthylcarbodiimid, N-Athyl-N′-(3-dimethylaminopropyl)-carbodiimid, N,N′-Carbonyldi-(2-methylimidazol) oder N,N′-(Dicarbonyl)-2-methylimidazol, Pentamethylenketen-N-cyclohexylimin, Diphenylketen-N-cyclohexylimin, ein Alkoxyacetylen, wie z.B. Methoxyacetylen und Äthoxyacetylen, ein 1-Alkoxy-1-chloräthylen, wie z.B. 1-Methoxy-1-chloräthylen und 1-Äthoxy-1-chloräthylen, ein Trialkylphosphit, wie z.B. Trimethylphosphit und Triäthylphosphit, Äthylpolyphosphate, Isopropylpolyphosphate, Phosphoroxychlorid, Thionylchlorid, Oxalylchlorid, Triphenylphosphin, das 2 - Äthyl - 7 - hydroxybenzisoxazoliumsalz, das innere Komplexsalz 2 - Äthyl-5 - m - sulfophenylisoxazoliumhydroxid, (Chlormethylen)dimethylammoniumchlorid, $SO_3$ - Hexamethylphosphoramid, $SO_3$ - N,N - dimethylformamid, Sulfonate, Trifluoressigsäureanhydrid, Diphenylphosphorsäureazid, Diphenylphosphorsäurecyanid und beliebige Mischungen davon; wobei man das genannte Dehydratisierungsmittel in einer Menge von 0,5 Mol bis 20 Mol und vorzugsweise 1 Mol bis 10 Mol pro Mol freie 1H-Tetrazol-1-essigsäure verwendet.

6. Verfahren nach Anspruch 2, 3, 4 oder 5, dadurch gekennzeichnet, daß man das 1,3,4-Thiadiazol-5-thiol oder sein Derivat in einer Menge im Bereich von 0,5 Mol bis 2 Mol und vorzugsweise 0,9 Mol bis 1,2 Mol pro Mol 1H-Tetrazol-1-essigsäure oder ihrem Derivat verwendet.

7. Verfahren nach Anspruch 2, 3, 4, 5 oder 6, dadurch gekennzeichnet, daß man die Reaktion bei einer Reaktionstemperatur im Bereich von −50°C bis 100°C und vorzugsweise −20°C bis 50°C durchführt.

8. Verfahren nach Anspruch 2, 3, 4, 5, 6 oder 7, dadurch gekennzeichnet, daß man die Reaktion in einem inaktiven oder inerten Reaktionsmedium durchführt.

9. Verfahren zur Herstellung einer Cephalosporinverbindung der Formel

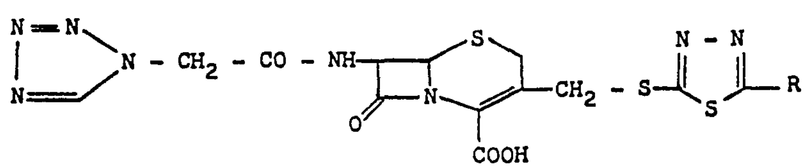

worin R ein Wasserstoffatom oder ein Methylrest ist, dadurch gekennzeichnet, daß man 7-Aminocephalosporansäure oder ihr Derivat mit dem 1,3,4-Thiadiazol-5-thiolester gemäß Anspruch 1 bei einer

Temperatur von −50°C bis 100°C und vorzugsweise 0°C bis 80°C durchführt.

10. Verfahren nach Anspruch 9, dadurch gekennzeichnet, daß man ein Derivat von 7-Aminocephalosporansäure verwendet, das aus der nachstehenden Gruppe ausgewählt ist:
die Salze von Alkalimetallen, wie z.B. Natrium und Kalium; die Salze von Erdalkalimetallen, wie z.B. Calcium und Magnesium; die Salze von stickstoffhaltigen organischen Basen, wie z.B. Trimethylamin, Triäthylamin, Pyridin, N-Methylpiperidin, N-Methylmorpholin; und die Ester, die man durch katalytische Reducktion oder chemische Reduktion oder unter anderen milden Bedingungen leicht freisetzen kann, wie z.B. der Toluolsulfonyläthylester, Benzylester, p-Nitrobenzylester, Phenacylester, Diphenylmethylester, Tritylester, t-Butylester, ein Alkyloxymethylester, wie z.B. Methoxymethylester und Athoxymethylester, Phenyloxymethylester, Benzoyloxymethylester, Acetyloxymethylester, 3,5-Di-(t-butyl-4-hydroxybenzylester und $\beta,\beta,\beta$-Trichloräthylester.

11. Verfahren nach Anspruch 9 oder 10, dadurch gekennzeichnet, daß man den 1,3,4-Thiadiazol-5-thiolester in einer Menge im Bereich von 0,5 Mol bis 2,0 Mol und vorzugsweise 0,9 Mol bis 1,2 Mol pro Mol 7-Aminocephalosporansäure oder ihrem Derivat verwendet.

12. Verfahren nach Anspruch 9, 10 oder 11, dadurch gekennzeichnet, daß man die Reaktion in einem inaktiven oder inerten Reaktionsmedium durchführt.

13. Verfahren nach Anspruch 9, 10, 11 oder 12, dadurch gekennzeichnet, daß man die Reaktion bei einem pH-Wert im Bereich von 1 bis 9 und vorzugsweise 3 bis 8 durchführt.

14. Verfahren nach Anspruch 9, 10, 11, 12 oder 13, dadurch gekennzeichnet, daß man die Reaktion in Gegenwart eines Promotors durchführt, der aus der nachstehenden Gruppe ausgewählt ist: eine Base, die ein Alkalimetallhydrogencarbonat, wie z.B. Natriumhydrogencarbonat und Kaliumhydrogencarbonat, ein Trialkylamin, wie z.B. Trimethylamin und Triäthylamin, Pyridin umfaßt; ein Salz einer anorganischen Säure, wie z.B. ein Chlorid, Bromid, Jodid, Thiocyanat oder Nitrat von Lithium, Natrium, Kalium oder Ammonium; eine Metallverbindung, wie z.B. Kupfer-(II)-chlorid, Kupfer-(II)-bromid, Kupfer-(II)-fluorid, Kupfer-(II)-nitrat, Kupfer-(I)-nitrat, Kupfer-(II)-sulfat, Kupfer-(I)-sulfat, Kupfer-(II)-borat, Kupfermetaborat, Kupfer-(II)-phosphat, Kupfer-(II)-cyanid, Kupfer-(I)-cyanid, Kupfer-(II)-formiat, Kupfer-(I)-formiat, Kupfer-(II)-acetat, Kupfer-(I)-acetat, Kupferpropionat, Kupfercitrat, Kupfer-(II)-tartrat, Kupfer-(I)-tartrat, Kupfer-(II)-benzoat, Kupfer-(I)-benzoat, Kupfer-(II)-salicylat, Kupfer-(I)-salicylat, Eisen-(III)-chlorid, Eisen-(II)-chlorid; eine Lewis-Säure, wie z.B. Titantetrachlorid, Titantetrabromid, Zirkontetrachlorid, Siliciumtetrachlorid, Zinntetrachlorid, Antimontrichlorid, Antimonpentachlorid, Bismuttrichlorid, Aluminiumtrichlorid, Aluminiumtribromid, Zinkdichlorid, Bortrifluorid, Bortrichlorid, Bortribromid; ein quaternäres Ammoniumsalz, wie z.B. Tetramethylammoniumchlorid, Tetraäthylammoniumbromid, Dimethyldiphenylammoniumchlorid, Triäthylbenzylammoniumbromid; und ein Tetraalkylphosphoniumhalogenid, wie z.B. Tetramethylphosphoniumchlorid, Tetraäthylphosphoniumbromid und Tetraphenylphosphoniumjodid; wobei man den genannten Promotor in einer Menge im Bereich von 0,1 Mol bis 10 Mol und vorzugsweise von 1 Mol bis 5 Mol pro Mol 7-Aminocephalosporansäure oder ihrem Derivat verwendet.

15. Zusammensetzung für ein schlafeinleitendes Anästhetikum oder für antibakterielle Zwecke, dadurch gekennzeichnet, daß sie einen 1,3,4-Thiadiazol-5-thiolester gemäß Anspruch 1 enthält oder daraus besteht.

## Revendications

1. 1,3,4-thiadiazole-5-thiol ester, caractérisé en ce qu'il a pour formule

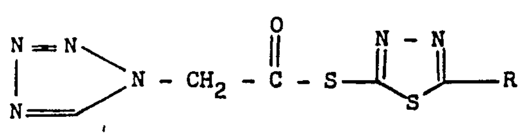

où R est un atome d'hydrogène ou un groupe méthyle.

2. Procédé de préparation du 1,3,4-thiadiazole-5-thiol ester selon la revendication 1, caractérisé en ce qu'il consiste à faire réagir un 1,3,4-thiadiazole-5-thiol de formule

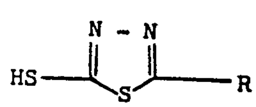

où R est un atome d'hydrogène ou un groupe méthyle, ou son dérivé, avec de l'acide 1H-tétrazole-1-acétique (en présence d'un agent déshydratant) ou son dérivé.

3. Procédé selon la revendication 2, caractérisé en ce qu'on utilise un dérivé du 1,3,4-thiadiazole-5-thiol, qui est choisi dans le groupe qui suit:
les sels d'un métal alcalin comme le sodium ou le potassium; les produits réactionnels avec un trialkylaluminium tels que le triméthylaluminium et le triéthylaluminium; les dérivés de silyle comme le

dérivé de triméthylsilyle et le dérivé de triethylsilyle; et les trifluoroacétates.

4. Procédé selon la revendication 2 ou 3, caractérisé en ce qu'un dérivé de l'acide 1H-tétrazole-1-acétique est utilisé, qui est choisi dans le groupe qui suit:
les halogénures acides comme le chlorure acide; les anhydrides acides mélangés avec un acide dialkylphosphorique tel que l'acide diméthylphosphorique et l'acide diéthylphosphorique, l'acide phénylphosphorique, l'acide diphénylphosphorique, l'acide dibenzylphosphorique, un acide phosphorique halogéné comme l'acide chlorophosphorique et l'acide bromophosphorique, un acide dialkylphosphoreux comme l'acide diméthylphosphoreux et l'acide diéthylphosphoreux, l'acide sulfureux, l'acide thiosulfurique, l'acide sulfurique, un acide alkylcarbonique comme l'acide méthylcarbonique et l'acide éthylcarbonique, un acide carboxylique aliphatique tel que l'acide pivalique, l'acide pentanoïque, l'acide isopentanoïque, l'acide 2-éthylbutanoïque, l'acide trichloroacétique et l'acide trifluoroacétique, un acide carboxylique aromatique comme l'acide benzoïque; l'anhydride acide symétrique de l'acide 1H-tétrazole-1-acétique, les amides acides avec l'imidazole, un imidazole substitué en position 4 comme le 4-méthylimidazole et le 4-éthylimidazole, le diméthylpyrazole, le triazole, le tétrazole, l'ammoniac, la méthylamine, la diméthylamine; les esters comme le méthyl ester, l'éthyl ester, le cyanométhyl ester, le méthoxyméthyl ester, le vinyl ester, le propargyl ester, le p-nitrophényl ester, le 2,4-dinitrophényl ester, le trichlorophényl ester, le pentachlorophényl ester, le méthanesulfonylphényl ester, le phénylazophényl ester, le phényl ester, le phénylthioester, le p-nitrophénylthioester, le 2,4-dinitrophénylthioester, le p-crésylthioester, le carboxyméthylthioester, le pyranyl ester, le pyridyl ester, le 8-quinolylthioester, l'ester avec la N,N-diméthylhydroxylamine, la 1-hydroxy-2-(1H)-pyridone, le N-hydroxysuccinimide ou le N-hydroxyphtalimide; les azides acides; et le nitrile.

5. Procédé selon la revendication 2 ou 3, caractérisé en ce que l'acide 1H-tétrazole-1-acétique libre est utilisé en présence d'un agent déshydratant qui est choisi dans le groupe suivant:
N,N - dicyclohexylcarbodiimide, N,N' - diisopropylcarbodiimide, N - cyclohexyl - N' - morpholinoéthylcarbodiimide, N - cyclohexyl - N' - (4 - diéthylaminocyclohexyl)carbodiimide, N,N' - diéthylcarbodiimide, N - éthyl - N' - (3 - diméthylaminopropyl)carbodiimide, N,N' - carbonyldi(2 - méthylimidazole), pentaméthylènecétène - N - cyclohexylimine, diphénylcétène - N - cyclohexylimine, un alcoxyacétylène comme le méthoxyacétylène et l'éthoxyacétylène, un 1-alcoxy-1-chloroéthylène comme le 1-méthoxy-1-chloroéthylène et le 1-éthoxy-1-chloroéthylène, un trialkyl phosphite comme le triméthyl phosphite et le triéthylphosphite, des polyphosphates d'éthyle, du polyphosphate d'isopropyle, de l'oxychlorure phosphoreux, du chlorure de thionyle, du chlorure d'oxalyle, de la triphényl phosphine, du sel de 2-éthyl-7-hydroxybenzisoxasolium, du sel interne d'hydroxyde de 2-éthyl-5-m-sulfophénylisoxazolium, du chlorure de (chlorométhylène)diméthylammonium, $SO_3$-hexaméthylphosphoramide, $SO_3$-N,N-diméthylformamide, sulfonates, anhydride trifluoroacétique, azide d'acide diphénylphosphorique, cyanure d'acide diphénylphosphorique et tout mélange; ledit agent déshydratant étant utilisé en une quantité de 0,5 mole à 20 moles et de préférence de 1 mole à 10 moles par mole de l'acide 1H-tétrazole-1-acétique libre.

6. Procéde selon l'une des revendications 2, 3, 4 ou 5, caractérisé en ce que le 1,3,4-thiadiazole-5-thiol ou son dérivé est utilisé en une quantité comprise entre 0,5 mole et 2 moles, et de préférence entre 0,9 mole et 1,2 moles par mole de l'acide 1H-tétrazole-1-acétique ou son dérivé.

7. Procédé selon l'une quelconque des revendications 2, 3, 4, 5 ou 6, caractérisé en ce que la réaction précitée est effectuée à une température comprise entre −50°C et 100°C et de préférence entre −20°C et 50°C.

8. Procédé selon l'une quelconque des revendications 2, 3, 4, 5, 6 ou 7, caractérisé en ce que la réaction précitée est effectuée dans un mileu réactionnel inactif.

9. Procédé de préparation d'un composé de céphalosporine de formule:

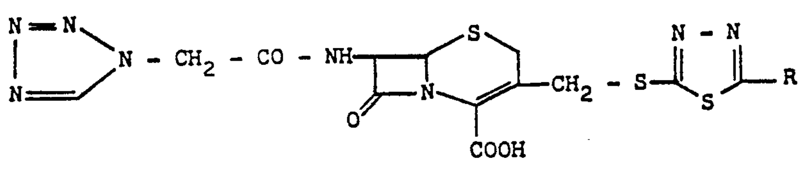

où R est un atome d'hydrogène ou un groupe méthyle, caractérisé en ce qu'il consiste à faire réagir de l'acide, 7-aminocéphalosporanique ou son dérivé avec le 1,3,4-thiadiazole-5-thiol ester selon la revendication 1, à une température de −50°C à 100°C et de préférence de 0°C à 80°C.

10. Procédé selon la revendication 9, caractérisé en ce qu'on utilise un dérivé de l'acide 7-aminocéphalosporanique qui est choisi dans le groupe qui suit:
les sels de métaux alcalins comme le sodium et le potassium; les sels de métaux alcalino-terreux comme le calcium et le magnésium; les sels de bases organiques contenant de l'azote comme la triméthylamine, la triéthylamine, la pyridine, la N-méthylpipéridine, la N-méthylmorpholine esters qui peuvent facilement être libérés par réduction catalytique ou réduction chimique ou dans d'autres

conditions modérées, comme le toluènesulfonyléthyl ester, le benzyl ester, le p-nitrobenzyl ester, le phénacyl ester, le diphénylméthyl ester, le trityl ester, le t-butyl ester, un alcoyloxyméthyl ester comme le méthoxyméthyl ester et l'éthoxyméthyl ester, le phényloxyméthyl ester, le benzoyloxyméthyl ester, l'acétyloxyméthyl ester, le 3,5-di-(t-butyl)-4-hydroxybenzyl ester et le $\beta,\beta,\beta$-trichloroéthyl ester.

11. Procédé selon la revendication 9 ou 10, caractérisé en ce que le 1,3,4-thiadiazole-5-thiol ester est utilisé en une quantité comprise entre 0,5 mole et 2,0 moles et de préférence entre 0,9 mole et 1,2 moles, par mole de l'acide 7-aminocéphalosporanique ou son dérivé.

12. Procédé selon l'une quelconque des revendications 9, 10 ou 11, caractérisé en ce que la réaction est effectuée dans un milieu réactionnel inactif.

13. Procédé selon l'une quelconque des revendications 9, 10, 11 ou 12, caractérisé en ce que la réaction précitée est effectuée à un pH compris entre 1 et 9 de préférence entre 3 et 8.

14. Procédé selon l'une quelconque des revendications 9, 10, 11, 12 ou 13, caractérisé en ce que la réaction précitée est effectuée en présence d'un promoteur qui est choisi dans le groupe qui suit: une base comprenant un hydrogénocarbonate d'un métal alcalin comme l'hydrogénocarbonate de sodium et l'hydrogénocarbonate de potassium, une trialkylamine comme la triméthylamine et la triéthylamine, la pyridine; un sel d'acide inorganique comme le chlorure, bromure, iodure, thiocyanate ou nitrate de lithium, sodium, potassium ou ammonium; un composé d'un métal tel que le chlorure cuprique, le bromure cuprique, le fluorure cuprique, le nitrate cuprique, le nitrate cuivreux, le sulfate cuprique, le sulfate cuivreux, le borate cuprique, le métaborate de cuivre, le phosphate cuprique, le cyanure cuprique, le cyanure cuivreux, le formiate cuprique, le formiate cuivreux, l'acétate cuprique, l'acétate cuivreux, le propionate de cuivre, le citrate de cuivre, le tartrate cuprique, le tartrate cuivreux, le benzoate cuprique, le benzoate cuivreux, le salicylate cuprique, le salicylate cuivreux, le chlorure ferrique, le chlorure ferreux; un acide de Lewis tel que le tétrachlorure de titane, le tétrabromure de titane, le tétrachlorure de zirconium, le tétrachlorure de silicium, le tétrachlorure d'étain, le trichlorure d'antimoine, le pentachlorure d'antimoine, le trichlorure de bismuth, le trichlorure d'aluminium, le tribromure d'aluminium, le dichlorure de zinc, le trifluorure de bore, le trichlorure de bore le tribromure de bore; un sel d'ammonium quaternaire comme le chlorure de tétraméthylammonium, le bromure de tétraéthylammonium, le chlorure de diméthyldiphénylammonium, le bromure de triéthylbenzyl-ammonium; et un halogénure de tétraalkylphosphonium comme le chlorure de tétraméthyl-phosphonium, le bromure de tétraéthylphosphonium et l'iodure de tétraphénylphosphonium; ledit promoteur étant utilisé en une quantité comprise entre 0,1 mole et 10 moles et de préférence entre 1 mole et 5 moles par mole de l'acide 7-aminocéphalosporanique ou son dérivé.

15. Composition dans des buts anesthésiques, antibactériens ou pour induire le sommeil, caractérisée en ce qu'elle se compose de ou contient un 1,3,4-thiadiazole-5-thiol ester selon la revendication 1.